Europäisches Patentamt
European Patent Office
Office européen des brevets

(19)

(11) Publication number: **0 501 511 A2**

(12) **EUROPEAN PATENT APPLICATION**

(21) Application number: **92103473.2**

(22) Date of filing: **28.02.92**

(51) Int. Cl.5: **C07H 19/04**, C07D 405/04, C07D 405/14, C07D 473/00, A61K 31/70, A61K 31/505, A61K 31/52

(30) Priority: **01.03.91 US 663504**

(43) Date of publication of application: **02.09.92 Bulletin 92/36**

(84) Designated Contracting States: **AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Kim, Choung Un**
**13 High Field Lane**
**Madison, Connecticut 06443(US)**
Inventor: **Martin, John C.**
**116 Leslie Drive**
**San Carlos, California 94070(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **Processes for preparation of nucleoside derivatives.**

(57) Improved processes for production of useful nucleoside derivatives of formula (VI)

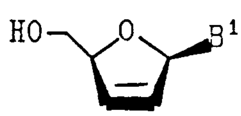

VI

wherein $B^1$ is a pyrimidinyl group of Formula XXI or a purinyl group of Formula XXII,

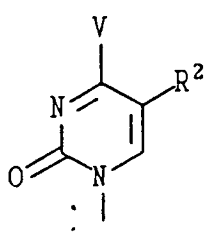

XXI

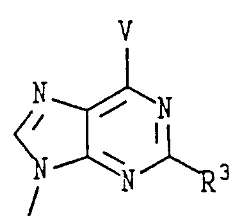

XXII

in which V is amino or hydroxy,
$R^2$ is hydrogen or methyl, and
$R^3$ is hydrogen or amino;

EP 0 501 511 A2

This invention describes new and useful chemical processes which represent a considerable improvement for the synthesis of nucleoside derivatives. These novel processes employ versatile intermediate tetrahydro furanyl compounds adapted to the manufacture of anti-cancer and anti-infective, and in particular, antiviral nucleoside compounds. One aspect of the usefulness of these processes concerns synthetic control of the appropriate stereochemistry in the final nucleosidic products. Synthetic processes involving chemical intermediates which confer stereospecificity, or at least stereoselectivity, are greatly preferred in manufacturing nucleosides and nucleoside derivatives compared to prior art processes requiring isomer separations.

The classical approaches to nucleoside derivative synthesis have employed variations comprising either the alteration of intact nucleosides or the attaching of a purine or pyrimidine base to a modified carbohydrate component. The tetrahydro furanyl reactants used in the processes of the present invention can be viewed as modified carbohydrate components whose chemical structure disposes them to facile conversion to stereochemically correct nucleosidic products.

The use of various furanyl-type synthetic intermediates have been described in the art.

Vial, et al., describes the use of thymine derivatives intermediates 1-3 in Nucleosides and Nucleotides, 9(2), 245-258(1990); as being incorporated

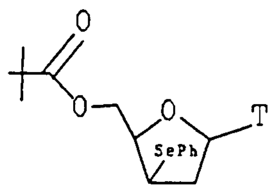

1

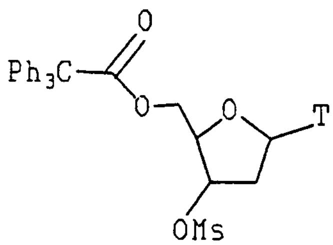

2

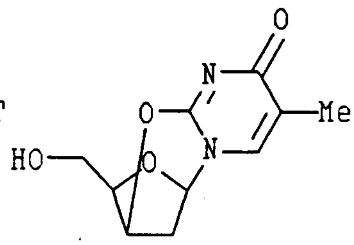

3

(T is the base thymine)

in processes to produce the anti-HIV agent 1-(2,3-dideoxy-β-D-glycero-pent-2-enofuranosyl)thymine (D4T).

Intermediates 4 and 5 are described by Wilson, et al,

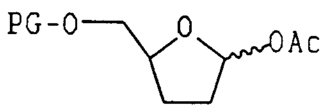

4

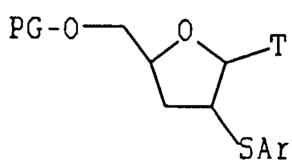

5

(PG is a protecting group; T is the base thymine)

in Tetrahedron Letters, 31/13, 1815-1818 (1990) as providing β-selectivity in processes for making D4T.

Intermediates 6 and 7 have been reported by Chu, et al,

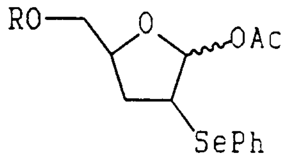 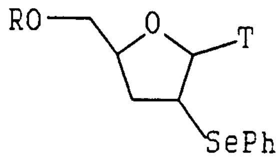

6 7

(R is a protecting group; T is the base thymine)

J. Org. Chem., 55/5, 1418-1420 (1990) in stereoselective processes for synthesizing 2',3'-dideoxy and 2',3'-dideoxy-2',3'-didehydronucleosides. Use of these previously described tetrahydrofuranyl intermediates however requires an isomeric separation step either of the intermediate itself prior to incorporation into the process or in subsequent steps in the process directed to a nucleoside derivative.

Sato in Japanese Kokai JO 2069-469-A, published 03/08/90, disclosed dihydrofurans 8 and 9 and the procedures for their preparation.

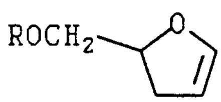 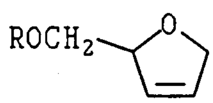

8 9

(R is benzyl or benzoyl)

These intermediates are disclosed in JO 2069-476-A, published 03/08/90 as starting materials for making pyrimidine dideoxynucleosides.

The processes of the present invention represent an advance over these prior art processes. Utilization of novel tetrafuranyl derivatives in these processes provides not only stereochemical specificity for the nucleoside products but product versatility as well, being adapted to processes for the synthesis of various nucleoside derivatives.

## Summary of The Invention

This invention comprises the incorporation of novel tetrahydrofuranyl compounds into processes for the synthesis of useful nucleoside derivatives. These synthetic processes produce various nucleoside derivative compounds with the intended stereochemical configurations. In addition to providing useful known nucleoside derivatives, the novel processes of this invention may also be utilized to synthesize new nucleoside derivatives.

## Detailed Description of the Invention

The following flow charts illustrate processes for preparation of novel synthetic intermediates (Scheme 1) and conversion of these intermediates into desired nucleoside derivatives (Scheme 2).

Scheme 1

Synthesis of Intermediates of Formula I

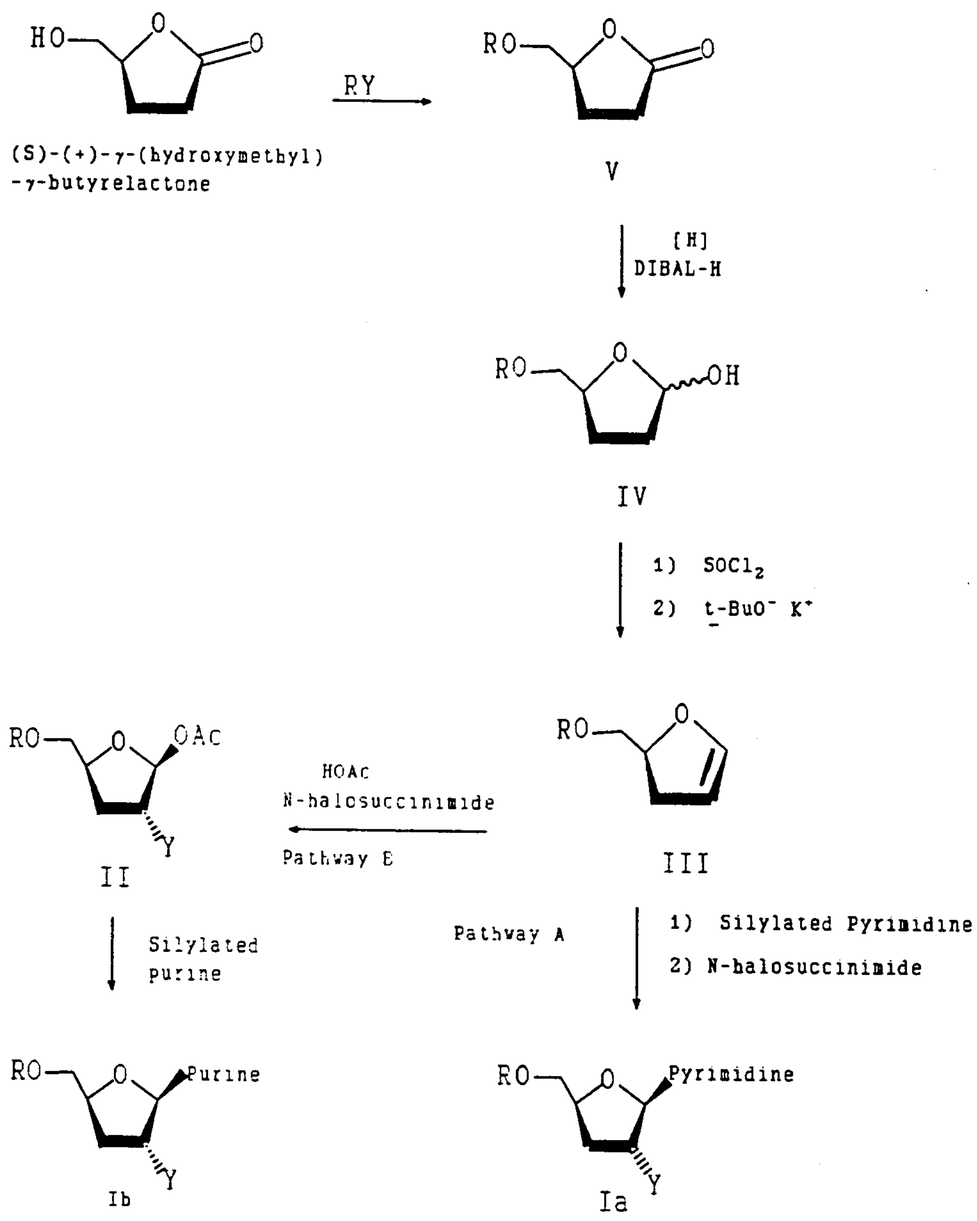

Scheme 2
Processes For Preparation of Nucleoside Derivatives

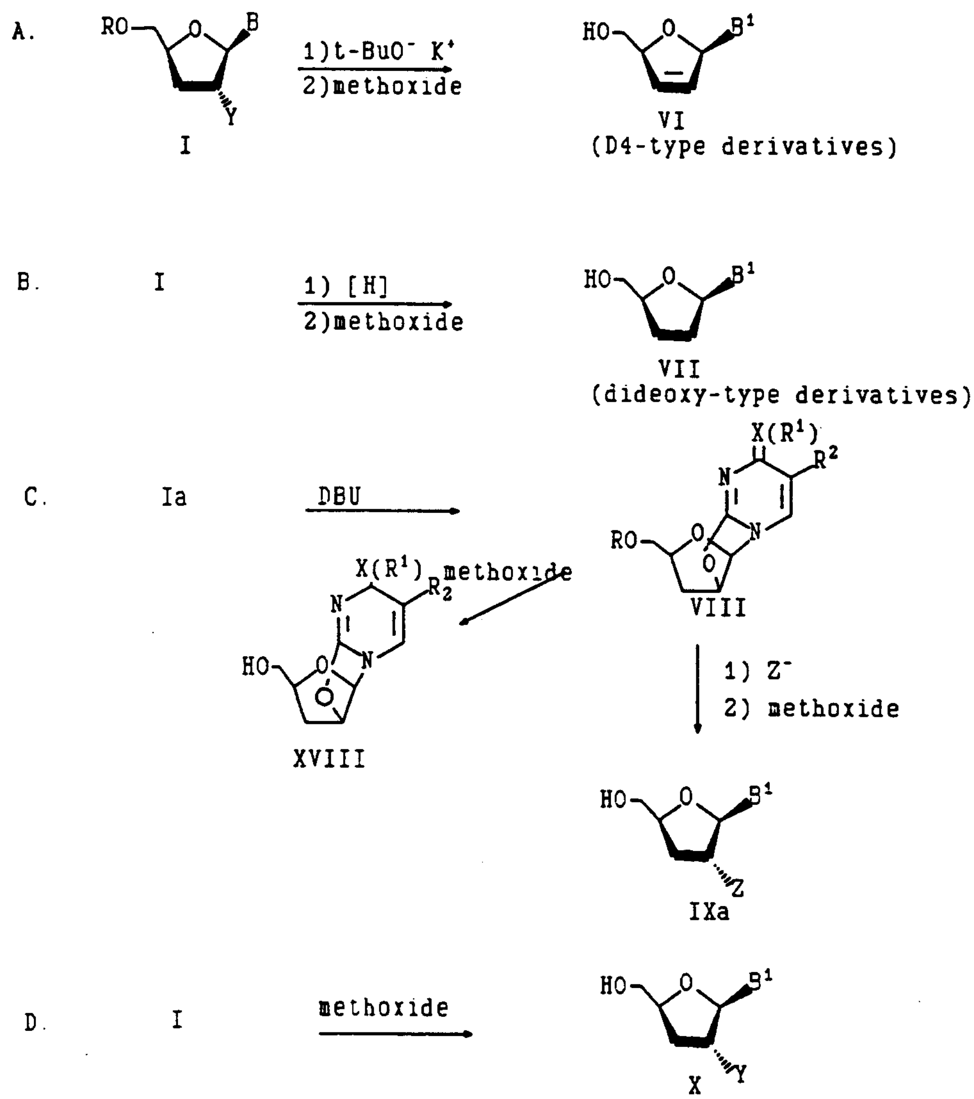


Scheme 1 basically outlines the synthesis of intermediate compounds of Formula I

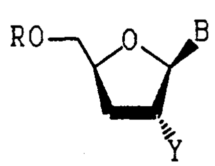

I

wherein R is a sterically bulky alkyl, aryl, silyl, or arylalkyl group such as pivaloyl, t-butydiphenylsilyl, trityl or 4-monomethoxytrityl and Y is chloro, bromo, or iodo. In Formula I, B is either a pyrimidinyl group of Formula XI or a purinyl group of Formula XII.

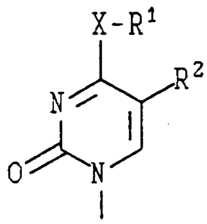
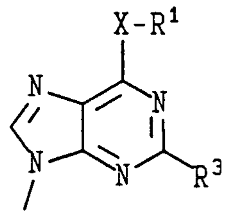

XI XII

In the heterocyclic moieties XI and XII, $XR^1$ is either amino, hydroxy or a protected amino or protected hydroxy; $R^2$ is hydrogen or methyl; and $R^3$ is hydrogen or amino. The XI and XII bases also exist in familiar tautomeric forms as appreciated by those of skill in the nucleoside art.

Formula I compounds then may comprise either pyrimidine modifications of Formula Ia,

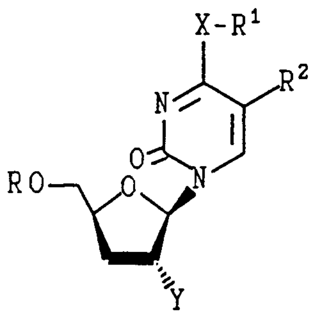

Ia

or purinyl modifications of Formula Ib.

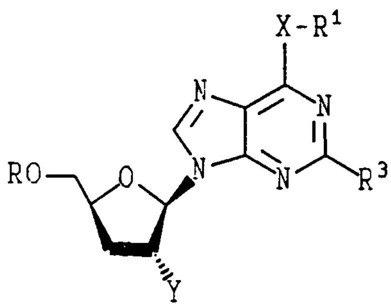

Ib

In structural Formulas Ia, Ib, XI, and XII; X refers to either -NH- or -O- linking moieties. $R^1$ can be either hydrogen or an organic synthetic protecting group of the type used to protect hydroxy and amino functional groups. For example, the primary alcohol or amino group on the pyrimidinyl or purinyl ring system may be protected as an ether or an ester when X is -O- or as an amide when X is -NH-. A preferred protection group is the trimethylsilyl group. The use of these and other organic synthetic protecting groups is well-known in the art and their use and chemistry is set forth in "Protective Groups in Organic Synthesis", T.W. Greene, John Wiley, New York, 1981.

The other substituent groups on the pyrimidinyl and purinyl rings are $R^2$ which can be hydrogen or methyl and $R^3$ which can be hydrogen or amino.

In Scheme 1, the initial step involves protecting the pendant hydroxy group of the starting (S)-(+)-butyrolactone by alkylation, acylation or silylation with RY to give the lactone of Formula V. Other known methods of protecting a pendant hydroxy group may also be employed. One preferred acylating agent is pivaloyl chloride. Lactone V is reduced with a selective hydride reducing agent such as diisobutylaluminum hydride (DIBAL-H) to give a mixture of $\alpha$- and $\beta$- anomers of compound IV. The anomeric mixture is

designated by use of the wavy line in structure IV. Dehydration of the lactol IV is effected by treatment with a halogenating agent such as thionyl chloride, followed by a strong base such as potassium t-butoxide or reaction of IV with oxalyl chloride followed by treatment with triethlylamine to give the dihydrofuran intermediate III. Depending on whether a purinyl (Ib) or pyrimidinyl (Ia) product of Formula I is desired; compound III is either reacted with a silylated pyrimidine, suitably protected, in the presence of an N-halosuccinimide such as N-iodosuccinimide (Pathway A) or is converted initially to the acetate intermediate II and then to the purinyl compound Ib (Pathway B).

The final steps shown in the process involve the use of either an activated pyrimidine or activated purine compound. These heterocyclic systems are activated synthetically by well-known methods in nucleoside chemistry which result in providing a silylated, acetylated or benzoylated base. In general, the heterocyclic base is activated by reaction of the pendant amino and hydroxy functionalities on the nucleus of the given base with silylating, acetylating, or benzoylating agents in standard methods familiar to one skilled in the art. Silylation is a preferred activating method. The $R^1$ group in purine bases is usually an acyl group, with benzoyl being preferred. The preferred activation of both purine and pyrimidine bases is achieved with silylation. Typical pyrimidine reagents used to provide Ia compounds comprise bis-2,4-trimethylsilylcytosine, bis-2,4-trimethylsilylthymine, and bis-2,4-trimethylsilyluracil. Some typical purine reagents which are reacted with the acetate compound II to provide Ib compound comprise 6-benzoylamino-9-trimethylsilylpurine, bis-6,9-trimethylsilylinosine, and tris-2,6,9-trimethylsilylguanine.

In summary, the synthetic process depicted in Scheme 1 comprises the following steps.

a) acylating (S)-(+)-γ-(hydroxymethyl)-γ-butyrolactone with RY to give a compound of Formula V;

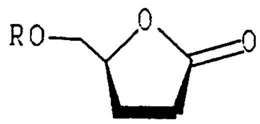

V

b) reducing compound V with diisobutylaluminum hydride or another selective hydride reducing agent to give an anomeric mixture of a compound of Formula IV;

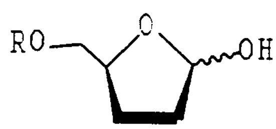

IV

c) converting the lactol IV to the corresponding dihydrofuranyl derivative III by treatment with thionyl chloride followed by potassium t-butoxide or with oxalyl chloride followed by triethylamine;

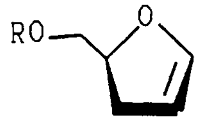

III

and

d) either following Pathway A and reacting compound III with a silylated pyrimidine derivative selected from bis-2,4-(trimethylsilyl)cytosine, bis-2,4-(trimethylsilyl)thymine, and bis-2,4-(trimethylsilyl)uracil; followed by successive treatment with an N-halosuccinimide such as N-iodosuccinimide and then aqueous bicarbonate solution to produce compound Ia

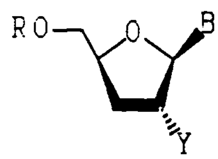

Ia

(B is a pyrimidinyl group of Formula XI as described supra); or following Pathway B and reacting compound III with acetic acid and then an N-halosuccinimide such as N-iodosuccinimide to give the acetate compound of Formula II.

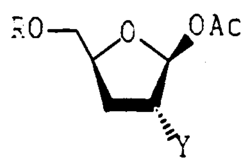

II

which is treated with a silylated purine derivative selected from 6-benzoylamino-9-trimethylsilylpurine, bis-6,9-trimethylsilylinosine, and tris-2,6,9-trimethylsilylguanine to produce compound Ib

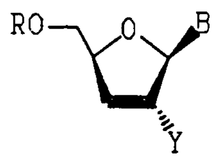

Ib

(B is a purinyl group of Formula XII as described supra).

In Scheme 2, the various processes comprising the convenient improved synthetic methods of the instant invention providing diverse nucleoside derivatives are set forth. These methods utilize the novel synthetic intermediates of Formula I whose syntheses are outlined in Scheme 1. In Scheme 2, B, R, $R^1$ and $R^2$ are as defined for Scheme 1 and $B^1$ is a purine or pyrimidine base of Formula XXII and XXI respectively.

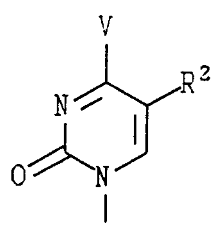 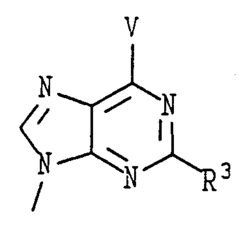

XXI XXII

in which V is amino or hydroxyl,
$R^2$ is hydrogen or methyl, and
$R^3$ is hydrogen or amino.

The difference between B and $B^1$ is that the protecting group on the amino or hydroxy group has been removed. The symbols $X(R^1)$ denoted in structures VIII and XVIII are intended to depict the oxy or imine groups; i.e.

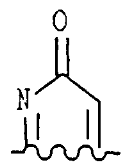

and

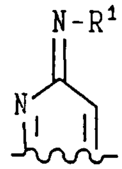

The symbol Z denotes a nucleophilic group selected from amino, azide, halo, hydroxy, and the like.

In Scheme 2, process A provides dideoxydidehydro riboside derivatives (D4-type) of Formula VI by treating intermediate I with a sterically bulky strong base such as potassium t-butoxide to give a compound VI precursor having Formula XVI.

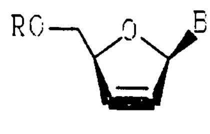

XVI

The desired D4-type nucleoside product of Formula VI is then obtained by removal of the protecting acyl group utilizing, for example, sodium methoxide in methanol. In this and the following processes, any protecting groups on the base moieties (XI and XII) are generally removed simultaneously by the pivaloyl cleavage reaction

(i.e. X-$R^1$ ----> V).

Process B provides dideoxyriboside derivatives (DD-type) of Formula VII by hydrogenolysis of the carbon-halide bond followed by removal of the protective groups as in Process A. The hydrogenolysis reaction can be accomplished using known standard methodology. A preferable hydrogenolysis technique employs hydrogen gas in the presence of a hydrogenolysis catalyst (such as palladium or platinum metals on a carbon support) at atmospheric pressures.

Process C produces pyrimidinyl riboside derivatives of Formula IXa wherein Z is selected from nucleophilic groups such as azide, hydroxy, amino, halo, and the like. Halo encompasses iodo, bromo and chloro. Chemically, Process C involves treatment of a pyrimidinyl intermediate of Formula Ia with 1,8-diazabicyclo [5,4,0] undec-7-ene (DBU) to give an anhydro compound of Formula VIII.

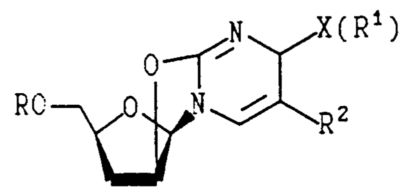

VIII

Compound VIII is then converted to compound 1Xa by reaction with an anionic nucleophile such as $^{-}NH_2$, $^{-}N_3$, $^{-}OH$, or halide; followed by treatment with sodium methoxide to remove the protective group. Alternatively compound VIII can be treated with methoxide to remove the protective group thereby producing the anhydro nucleoside derivative of Formula XVIII.

Process D yields a halo-substituted riboside derivative of Formula X by removal of the protective group of compound I. Again, a preferred method utilizes sodium methoxide.

Another aspect of the present invention is the provision of novel ribosidyl derivatives which can be obtained conveniently from these new processes. Examples of compounds which can be employed as anti-infective agents and which are also envisioned as antiviral substances are compounds of Formula IX with

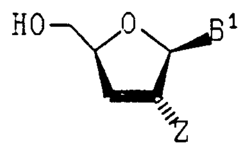

IX

$B^1$ and Z as defined hereinabove. Two specific novel IX compounds are

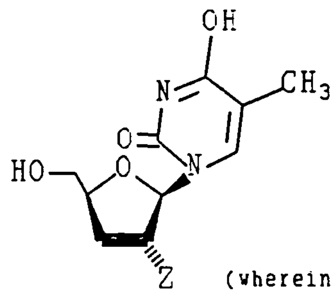

(wherein Z = $NH_2$, OH)

Description of Specific Embodiments

The processes of this invention whereby product nucleoside derivatives and furanyl intermediates are prepared are illustrated in greater detail by the following examples directed to preferred embodiments of the hereinabove described process steps of Schemes 1 and 2. These examples, however, should not be construed as limiting the scope of the present invention in any way.

I. Preparation of the Intermediates

Example 1

(S)-γ-Pivaloyloxymethyl-γ-butyrolactone (V)

To a solution of (S)-γ-hydroxymethyl-γ-butyrolactone[1](40 g, 0.34M) in pyridine (200 mL) was added pivaloyl chloride (46 g, 0.38M) and the mixture was heated at 50 °C for 5 h under nitrogen. The reaction was cooled to room temperature and MeOH (50 mL) was added. The mixture was then concentrated in vacuo, taken up in $CH_2Cl_2$-water. The $CH_2Cl_2$ was washed with water, 30% $H_3PO_4$, brine, and dried over $MgSO_4$. After removal of the solvent under reduced pressure, the residual oil was chromatographed on silica gel using $CH_2Cl_2$ as eluent to give 2 (48 g, 70%) as a colorless oil: $^1$H NMR ($CDCl_3$) δ 1.15 (s, 9H), 1.9-2.6 (m, 4H), 4.09 (dd, J = 4.8, 12.3 Hz, 1H), 2.29 (dd, J = 3.3, 12.3 Hz, 1H), 4.7-4.75 (m, 1H).

Example 2

2-(R,S)-Hydroxy-5-(S)-(pivaloyloxymethyl)tetrahydrofuran (IV)

To a solution of (S)-γ-pivaloyloxymethyl-γ-butyrolactone (44.5 g, 0.22M) in ether (1L) and THF (150 mL) at -70 °C, under nitrogen, was added 1M diisobutylaluminum hydride (DIBAL-H) in THF (250 mL, 0.25M) over 90 min. After stirring at -70 °C for 2 h, MeOH (80 mL) was added and the reaction was warmed to room temperature and a 60% solution of potassium sodium tartrate (800 mL) was added. The mixture was stirred for 2 h, and the organic layer was separated, dried over $MgSO_4$, and concentrated in vacuo. The residual oil was chromatographed on silica gel using $CH_2Cl_2$-5% MeOH as eluent to give IV (37 g, 82%) as a colorless oil: $^1$H NMR ($CDCl_3$) δ 1.15 and 1.17 (s, 9H), 1.6-2.2 (m, 4H), 2.78 and 2.84 (broad s, 1H), 3.9-4.4 (m, 3H), 5.47 (d, J = 1.9 Hz, 0.5H), 5.55 (d, J = 3.9 Hz, 0.5H).

[1] Prepared according to the literature procedure: M. Taninguchi, K. Koga, S. Yamada, Tetrahedron, 30, 3547 (1974).

Example 3

(S)-5-Pivaloyloxymethyl-4,5-dihydrotetrahydrofuran (III)

To a solution of the lactol IV (5 g, 24.7 mmol) in $CH_2Cl_2$ (75 mL) was added thionyl chloride (6 g, 50 mmol) under nitrogen. After stirring at 23°C for 2 h, volatiles were removed in vacuo. The oily residue was dissolved in toluene and evaporated to dryness to give the chloro compound as a colorless oil. This material was used for the next step without further purification.

To a solution of the chloro compound obtained from the lactol IV (5 g, 24.5 mmol) by the procedure described above, in THF (60 mL) at -70°C was added potassium tert-butoxide (2.9 g, 25 mmol) portionwise under nitrogen. After stirring at -70°C for 1 h, the reaction was quenched by addition of acetic acid (5 mL) and the mixture was warmed to room temperature. The reaction solution was evaporated in vacuo and the residual oil was taken up in $CH_2Cl_2$, washed with aqueous $NaHCO_3$, dried over $MgSO_4$, and evaporated to dryness. The residual liquid was distilled to give III (2.7 g, 60%), bp 60-68°/4 Torr, as a colorless oil: NMR ($CDCl_3$) δ 1.18 (s, 9H), 2.28-2.39 (m, 1H), 2.60-2.75 (m, 1H), 4.08 (dd, J=6.0, 11.7 Hz, 1H), 4.16 (dd, J=4.5, 11.7 Hz, 1H), 4.65-4.75 (m, 1H), 4.85 (dd, J=2.4, 4.8 Hz, 1H), 6.25 (dd, J=2.4, 4.8 Hz, 1H); $^{13}C$ NMR (50.3 MHz, $CDCl_3$) δ 27.369, 31.662, 44.806, 66.146, 77.736, 78.756, 99.423, 145.772.

| | | |
|---|---|---|
| Anal. Calcd. for $C_{10}H_{16}O_3$: | C, 65.19; | H, 8.75. |
| Found: | C, 65.51; | H, 8.57. |

Example 4

5'-0-Pivaloyl-2'-iodo-2',3'-dideoxy-5-methyluridine (Ia)

To a suspension of thymine (1.5 g, 12 mmol) in hexamethyldisilazane (40 mL) was added chlorotrimethylsilane (0.2 mL) and the mixture was heated at reflux with exclusion of moisture for 15 h. The resulting clear solution was evaporated in vacuo. The residual oil was dissolved in xylene (20 mL) and concentrated to dryness in vacuo. The residual clear oil was dissolved in THF (40 mL) and dihydrofuran III (1.84 g, 10 mmol) was added. A solution of N-iodosuccinimide (2.25 g, 10 mmol) in THF (20 mL) was added at -15°C dropwise and the resulting yellow solution was allowed to stir for 90 min at -15°C. The reaction was quenched by addition of aqueous $NaHCO_3$ and THF was concentrated under reduced pressure. The residual oil was taken up in ether, washed with water, aqueous sodium bisulfite, dried over $MgSO_4$, and concentrated in vacuo to give Ia (4.1 g, 94%) as a yellow oil: $^1H$ NMR ($CDCl_3$) δ 1.18 (s, 9H), 1.87 (s, 3H), 2.2-2.4 (m, 2H), 4.25 (dd, J=3.3, 12.0 Hz, 1H), 4.2-4.3 (m, 1H), 4.39 (dd, J=4.5, 12.0 Hz, 1H), 6.18 (d, J=3.9 Hz, 1H), 7.21 (s, 1H).

Example 5

(2S,3R,5S)-2-Acetoxy-3-iodo-5-pivaloyloxymethyl tetrahydrofuran (II)

To a solution of the dihydrofuran III (710 mg. 3.85 mmol) in $CH_2Cl_2$ (10 mL) at -20°C, under nitrogen was added acetic acid (1.2 g, 20 mmol) followed by N-iodosuccinimide (877 mg, 3.85 mmol). After stirring at -20°C for 60 min., the reaction was diluted with ether (50 mL), washed with aqueous $NaHCO_3$, and dried over $MgSO_4$. Evaporation of the dried solvents gave the iodo acetate intermediate II as a slightly yellow oil: $^1H$ NMR ($CDCl_3$) δ 1.16 (s, 9H), 1.99 (s, 3H), 2.2-2.35 (m, 2H), 4.17 (d, J=4.2$H_2$, 2H), 4.25 (d, J-4.5 $H_2$, 1H), 4.65 (m, 1H), 6.38 (s, 1H). This material was used for further chemical transformation without purification.

Example 6

(2R,3R,5S)-6-N-Benzoyl-9-(tetrahydro-2-iodo-5-pivaloyloxymethyl-2-furanyl)adenine (Ib)

To a suspension of 6-N-benzoyladenine (87 mg, 3.5 mmol) in hexamethyldisilazine (15 mL) was added chlorotrimethylsilane (0.3 mL) and ammonium sulfate (30 mg) and the mixture was heated at 135°C for 18 h under nitrogen. The resulting clear solution was evaporation in vacuo with exclusion of moisture. The residual oil was dissolved in xylene (20 ml) and concentrated to dryness in vacuo. This residual clear oil

and the iodo acetate intermediate II obtained by the procedure described above was dissolved in 1,2-dichloroethane (10 mL). A solution of 1.0 M tintetrachloride in $CH_2Cl_2$ (3.2 mL) was added dropwise at 20°C and the resulting yellow solution was allowed to stir for 60 min at -20°C then for 2 h without the cooling bath. The reaction was quenched by the addition of aqueous $NaHcCO_3$ and diluted with $CH_2Cl_2$.The mixture was filtered and the organic phase was separated, dried over $MgSO_4$, and concentrated to dryness. The residual oil was chromatographed on silica gel using $CH_2Cl_2$ -5% MeOH as eluent to given Ib (650 mg 43%) as a white foam: $^1$H NMR ($CDCl_3$) δ 1.67 (s, 9H), 2.4-2.6 (m, 2H), 4.35-4.4 (m, 2H), 4.78 (m, 1H), 5.11 (m, 1H), 6.46 (d, J=3.0 $H_2$, 1H), 7.4-7.6 (m, 3H), 8.0 (d, J=3.0 $H_2$, 2H), 8.18 (s, 1H), 8.98 (s, 1H).

By appropriate modification of reagents and using the experimental procedures set forth above, additional Formula I intermediates can be prepared.

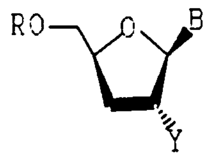

I

II. Preparation of Product Nucleoside Derivatives

Process A

Example 7

1-(2,3-Dideoxy-β-D-glycero-pent-2-enofuranosyl) thymine(D4T; VI)

To a solution of compound Ia (prepared in Example 4; 4.1 g, 11.3 mmol) in THF (25 mL) at 0°C was added a solution of potassium t-butoxide (1.7 g, 14.6 mmol) in THF (5 mL) over 3 min and the mixture was stirred for 30 min without the cooling bath under nitrogen. The reaction was quenched with aqueous $NH_4Cl$, and the mixture was extracted with ether (200 mL). The organic phase was washed with brine, dried over $MgSO_4$. After removal of the solvent, the residual oil was chromatographed on silica gel using $CH_2Cl_2$-3% MeOH as eluent to give 1-(5-0-Pivaloyl-2,3-dideoxy-β-D-glycero-pent-2-enofuranosyl)thymine (pivaloyl D4T 950 mg, 33%) as a white solid, readily crystallized in ether-$CH_2Cl_2$: mp 203°C (lit[2]206°C); NMR ($CDCl_3$) δ 1.18 (s, 9H), 1.89 (s, 3H), 4.14 (dd, J=5.0, 12.2 Hz, 1H), 4.38 (dd, J=3.7, 12.2 Hz, 1H), 5.03 (m, 1H), 5.92 (m, 1H), 6.27 (m, 1H), 6.94 (m, 1H), 7.14 (d, J=1.2 Hz, 1H), 8.35 (broad s, 1H).

To a solution of the pivaloyl D4T (800 mg, 2.6 mmol) in MeOH (30 mL) was added sodium methoxide (560 mg, 10.4 mmol) in MeOH (10 mL) and the mixture was allowed to stir for 4 h at room temperature. The reaction mixture was then treated with Dowex 50X8-200 mesh ion-exchange resin until the pH of the solution becomes 7.0. The mixture was filtered and the filtrate was evaporated to dryness in vacuo to give D4T product (600 mg, 100%) as a white solid: mp 152°C; $^1$H NMR ($D_2O$) δ 1.81 (s, 3H), 3.74 (d, J=3.0 Hz, 2H), 4.95 (broad s, 1H), 5.91 (d, J=5.9 Hz, 1H), 6.42 (d, J=5.9 Hz, 1H), 6.90 (broad s, 1H), 7.58 (s, 1H).

This NMR was identical with that of authentic D4T.

Process B

Example 8

2',3'-dideoxyadenosine (DDA; VII)

A solution of the (2R,3R,5S)-6-N-benzoyl-9-(tetrahydro-2-iodo-5-pivaloyloxymethyl-2-furanyl) adenine (Example 6; 150 mg. 0.37mmol) in EtOH-water (9:1) (10 mL) was hydrogenated in the presence of 10% palladium on carbon (80 mg) at atmospheric pressure for 6 h. The catalyst was filtered through celite and was washed with hot EtOH (5 mL). The combined solvents were evaporated to dryness. The residual oil

[2] J.-M. Vial, P. Agback, and J. Chattopadhyaya, Nucleosides & Nucleotides, 9, 245 (1990).

was then chromatographed on silica gel using $CH_2Cl_2$-3% MeOH as eluent to give 6-N-benzoyl-5'-O-pivaloyl-2',3'-dideoxyadinosine (69 mg, 59%) as a white amorphous powder: $^1H$ NMR ($CDCl_3$) δ 1.14 (s, 9H), 1.9-2.6 (m, 4H), 4.27 (d, J=4.5H$_2$,2H), 4.4 (m, 1H), 6.08 (q, J=6.9, 3.3H$_2$, 1H), 7.91 (s, 1H), 8.04 (s, 1H).

To a solution of this pivaloyl DDA (69 mg, 0.16 mmol) in MeOH (2 mL) at 0°C was added 25% sodium methoxide in MeOH (0.5 mL). After stirring at 25°C for 8 h, the solution was carefully neutralized to pH = 7.0 by the addition of 1N-HCl. All volatiles were removed in vacuo and the residue was purified by $C_{18}$ reverse phase column under 8 psi pressure using MeOH-water (1:1) as eluent to give DDA (15 mg, 39%) as amorphous white powder: $^1H$ NMR (D20) 1.9-2.6 (m, 4H), 3.59 (q, J=12.6, 5.1 H$_2$, 1H), 3.78 (q, J=12.6, 3.OH$_2$, 1H), 4.27 (m, 1H), 6.08 (q, J=6.9, 3.3 H$_2$, 1H), 7.91 (s, 1H), 8.04 (s, 1H).
This NMR was identical with that of DDA supplied by the Bristol-Myers Squibb Company, Industrial Division.

Process C

Example 9

2,2'-Anhydro-1-(5'-O-pivaloyl-3'-deoxy-β-D-arabinofuranosyl)thymine VIII)

To a solution of compound Ia (prepared in Example 4: 5.0 g, 11.5 mmol) in $CH_2Cl_2$ (100 mL) at 0°C, under nitrogen, was added 1,8-diazabicyclo [5,4,0] undec-7-ene (4.2 g, 28 mmol). After stirring at 0°C for 1 h, the reaction mixture was washed with 10% $H_3PO_4$, brine, dried over $MgSO_4$ and concentrated in vacuo. The residual oil was chromatographed on silica gel using $CH_2Cl_2$-5% MeOH as eluent to give compound VIII (2.6 g, 48%) as a white powder: $^1H$ NMR ($CDCl_3$) δ 1.18 (s, 9H), 1.95 (s, 3H), 2.3-2.6 (m, 2H), 3.90 (m, 2H), 4.53 (m, 1H), 4.54 (m, 1H), 6.04 (d, J=5.7 Hz, 1H), 7.18 (s, 1H).

Example 10

2,2'Anhydro-1-(3-deoxy-β-D-arabinofuranosyl)thymine (XVIII).

To a solution of Compound VIII (800 mg, 2.6 mmol) in MeOH (10 mL) was added 1N $CH_3ONa$ in MeOH (10 mL) and the mixture was stirred at room temperature for 4 h under nitrogen. The reaction mixture was then treated with Dowex 50X8-200 mesh ion-exhange resin until the pH of the solution becomes 7.0. The mixture was filtered and the filtrate was evaporated to dryness. The residual oil was purified by $C_{18}$ reverse phase column using water-20% MeOH as eluent under 8 psi pressure to give Compound XVIII (200 mg, 34%) as a white powder: UV max ($H_2O$)λ 266 nm (ε 6500); $^1H$ NMR ($CD_3OD$) δ 1.69 (s, 3H), 2.2-2.4 (m, 2H), 3.06 (dd, J=5.3, 12.6 Hz, 1H), 3.19 (dd, J=4.5, 12.6 Hz, 1H), 4.22 (m, 1H), 5.31 (dd, J=4.7, 5.7 Hz, 1H), 5.99 (d, J=5.7 Hz, 1H), 7.44 (s, 1H).

Example 11

2'α-Azido-2',3'-dideoxy-5-methyluridine (1Xa, X=$N_3$

To a solution of compound VIII (400 mg, 1.3 mmol) in DMF (4 mL) was added sodium azide (253 mg, 3.9 mmol) in water (1 mL) and the mixture was heated at 125°C for 16 h under nitrogen. The reaction was cooled to room temperature and volatiles were removed in vacuo. The residual oil was taken up in ether (50 mL) and washed with water, brine, dried over $MgSO_4$, and evaporated to dryness. The crude material was chromatographed on silica gel using $CH_2Cl_2$-5% MeOH as eluent to give 5'-O-pivaloyl-2'-azido-2',3'-dideoxy-5-methyluridine (140 mg, 32%) as a colorless oil: $^1H$ NMR ($CDCl_3$) δ 1.20 (s, 9H), 1.91 (s, 3H), 1.9-2.1 (m, 2H), 4.23 (dd, J=2.7, 12.4 Hz, 1H), 4.28 (m, 1H), 4.39 (dd, J=4.9, 12.4 Hz, 1H), 4.47 (m, 1H), 5.73 (d, J=2.0 Hz, 1H), 7.31 (s, 1H), 9.82 (broad s, 1H); $^{13}C$ NMR (50.3 MHz, $CDCl_3$) δ 178.870, 164.922, 151.051, 135.235, 111.338, 91.466, 79.029, 64.109, 64.227, 39.149, 31.841, 27.414, 12.773: IR (neat) $\gamma_{max}$ 2110 $cm^{-1}$.

To a solution of 5'-O-pivaloyl-2'-azido-2',3'-dideoxy-5-methyluridine (120 mg. 0.36 mmol) in MeOH (5 mL) was added 1N $CH_3ONa$ in MeOH (1.5 mL) and the solution was stirred at room temperature for 4 h. The reaction was quenched with 1N HCl and adjusted the pH of the solution to 8.0. Volatiles were removed in vacuo and the residual oil was chromatographed on silica gel using $CH_2Cl_2$-5% MeOH as eluent to give the 2'-azido-dideoxy product (60 mg, 67%) as a white solid: mp 140°C; UV λ max ($H_2O$) 268 nm (ε 7911);

IR (KBr) $\gamma_{max}$ 2135 $cm^{-1}$; $^1H$ NMR ($CD_3OD$) $\delta$ 1.61 (s, 3H), 1.70 (ddd, J=2.4, 5.7, 13.6 Hz, 1H), 2.0 (ddd, J=6.5, 9.9, 13.6 Hz, 1H), 3.43 (dd, J=3.0, 12.9 Hz, 1H), 3.71 (dd, J=2.7, 12.9 Hz, 1H), 4.09 (m, 2H), 5.58 (d, J=1.8 Hz, 1H), 7.77 (s, 1H); $^{13}C$ NMR (50.3 Hz, $CD_3OD$) $\delta$ 166.823, 152.549, 137.977, 111.168, 91.697, 83.193, 67.974, 62.913, 31.637, 12.742.

| Anal. Calcd. for $C_{10}H_{13}N_5O_4 \cdot 1/2\ H_2O$ | | | |
|---|---|---|---|
| | C, 43.47; | H, 5.07; | N, 25.36. |
| Found: | C, 43.75; | H, 4.86; | N, 24.83. |

Process D

Example 12

By removal of the protecting groups of intermediates of Formula I by application of standard cleavage processes such as those described in the foregoing examples, products of Formula X may be obtained. This process is particularly of value for Compound X products wherein $B^1$ comprises purine bases.

Similarly, other nucleoside derivatives can be obtained by ready adaptation of these processes in a manner familiar to one skilled in the synthesis of nucleoside products.

**Claims**

**1.** A process for preparation of a compound of Formula VI

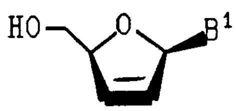

VI

wherein $B^1$ is a pyrimidinyl group of Formula XXI or a purinyl group of Formula XXII,

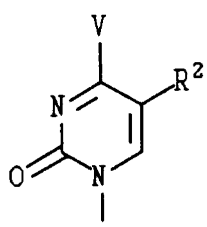

XXI

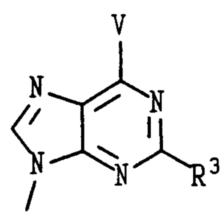

XXII

in which V is amino or hydroxy,
$R^2$ is hydrogen or methyl, and
$R^3$ is hydrogen or amino;
the process comprising the steps of
a) treating a compound of Formula I

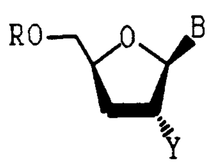

I

wherein R is a group selected from pivaloyl, t-butyldiphenylsilyl, trityl and 4-monomethoxytrityl; Y is selected from chloro, bromo and iodo; and B is a pyrimidinyl group of Formula XI or a purinyl group of Formula XII,

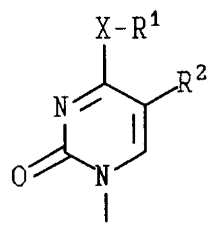

XI

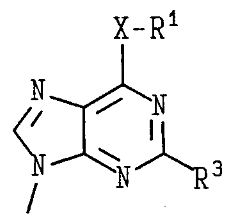

XII

in which X is -NH- or -O-;
$R^1$ is hydrogen or a protecting group;
$R^2$ is hydrogen or methyl; and
$R^3$ is hydrogen or amino;
with potassium t-butoxide to give a compound of Formula XVI;

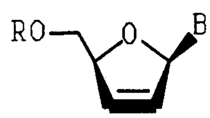

XVI

and
b) removing the protective groups by treating compound XVI with sodium methoxide to yield compound VI.

**2.** A process for preparation of a compound of Formula VII

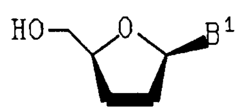

VII

wherein $B^1$ is a pyrimidinyl group of Formula XXI or a purinyl group of Formula XXII,

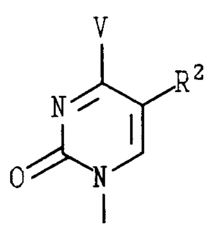

XXI

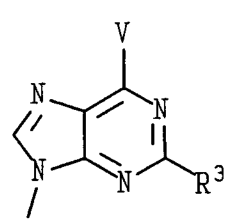

XXII

in which V is amino or hydroxy,
$R^2$ is hydrogen or methyl, and
$R^3$ is hydrogen or amino;

the process comprising the steps of

a) reacting a compound of Formula I

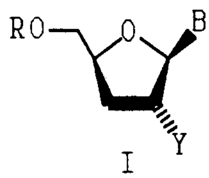

I

wherein R is a group selected from pivaloyl, t-butyldiphenylsilyl, trityl and 4-monomethoxytrityl; Y is selected from chloro, bromo, and iodo; and B is a pyrimidinyl group of Formula XI or a purinyl group of Formula XII

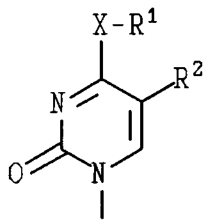

XI

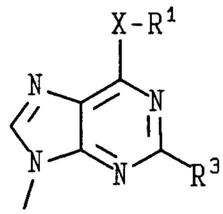

XII

in which X is -NH- or -O-;

$R^1$ is hydrogen or a protecting group;

$R^2$ is hydrogen or methyl; and

$R^3$ is hydrogen or amino;

with hydrogen gas in the presence of a hydrogenolysis catalyst at atmospheric pressure to yield compound XVII;

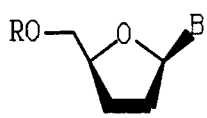

XVII

and

b) removing the protective groups by treating compound XVII with sodium methoxide to yield compound VII.

**3.** A process for the preparation of a compound of Formula IX

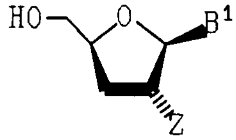

IX

wherein $B^1$ is a pyrimidinyl group of Formula XXI

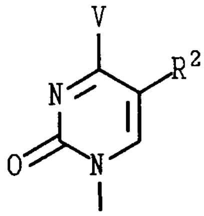

XXI

in which V is amino or hydroxy;
$R^2$ is hydrogen or methyl and
Z is amino, azido or hydroxy;
the process comprising the steps of
a) treating a pyrimidinyl compound of Formula Ia,

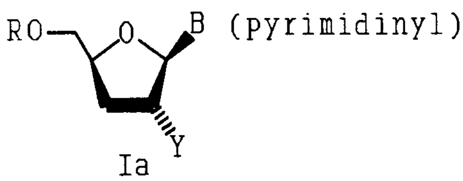

Ia

wherein R is a group selected from pivaloyl, t-butyldiphenylsilyl, trityl and 4-monomethoxytrityl; Y is selected from chloro, bromo, and iodo; and B is a pyrimidinyl group of Formula XXI

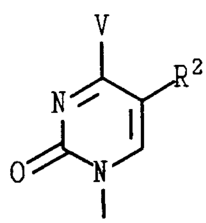

XXI

in which V is amino or hydroxy; and
$R^2$ is hydrogen or methyl;
with 1,8-diazabicyclo[5,4,0]undec-7-ene (DBU) to give an anhydro compound of Formula VIII;

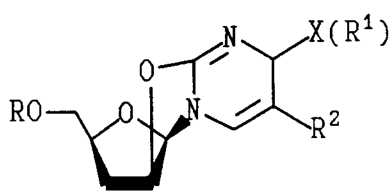

VIII

and
b) reacting compound VIII with an anionic species selected from $^-N_3$, $^-NH_2$ or $^-OH$; followed by treatment with sodium methoxide to give compound IX.

**4.** A process for preparation of a compound of Formula X

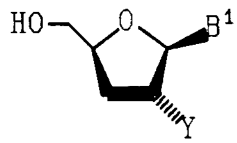

X

wherein B is a pyrimidinyl group of Formula XXI or a purinyl group of Formula XXII,

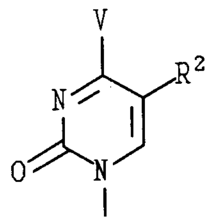 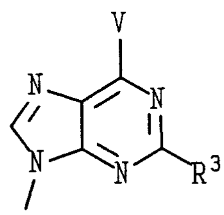

XXI XXII

in which V is amino or hydroxy; and
$R^2$ is hydrogen or methyl, and
$R^3$ is hydrogen or amino;
the process comprising the treatment of a compound of Formula I

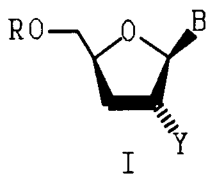

I

wherein R is a group selected from pivaloyl, t-butyldiphenylsilyl, trityl, and 4-monomethoxytrityl; Y is selected from chloro, bromo and iodo; and B is a pyrimidinyl group of Formula XI or a purinyl group of Formula XII,

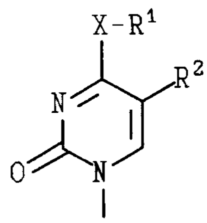 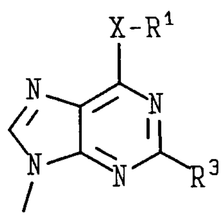

XI XII

in which X is -NH- or -O-;
$R^1$ is hydrogen or a protecting group;
$R^2$ is hydrogen or methyl; and
$R^3$ is hydrogen or amino;
with sodium methoxide to give compound X.

**5.** A compound of Formula IX of claim 3 in which X is amino.

**6.** The compound of claim 5, 2'-α-Amino-2',3'-dideoxy-5-methyluridine.

**7.** A compound of Formula IX of claim 3 in which X is hydroxy.

**8.** The compound of claim 7, 3'-Deoxy-5-methyluridine.

**9.** A pharmaceutical composition comprising at least one compound of Formula IX as defined in one of the claims 3 and 5 to 8, optionally in combination with a pharmaceutical acceptable carrier or diluent.

**10.** A process for preparing a pharmaceutical composition containing at least one compound of Formula IX as defined in one of the claims 3 and 5 to 8, characterized in that an effective amount of at least one compound of Formula IX is provided in a form suitable for administration, optionally in combination with a pharmaceutically acceptable carrier or diluent.

**11.** The use of a compound of Formula IX as defined in one of the claims 3 and 5 to 8 for preparing a pharmaceutical composition for the treatment of cancer and/or infections.